# EUROPÄISCHE PATENTANMELDUNG

(11) **EP 4 477 312 A1**
(43) Veröffentlichungstag der Anmeldung: **18.12.2024**
(21) Anmeldenummer: 24169783.8
(22) Anmeldetag: 11.04.2024
(51) Int. Cl.: B01L 1/02, G01N 17/00, F24F 6/18, C12M 1/04

(54) **KLIMASCHRANK**

(30) Priorität: 13.06.2023 DE 102023115363
(71) Anmelder: Binder GmbH, 78532 Tuttlingen (DE)
(72) Erfinder: BUSCHLE, Jochen, 78532 Nendingen (DE); KOSSMANN, Robin, 78532 Tuttlingen (DE)
(74) Vertreter: Westphal, Mussgnug & Partner Patentanwälte mbB

(57) **Zusammenfassung**

Bereitgestellt wird ein Klimaschrank (1) mit einem Außenkessel (10), mit einem innerhalb des Außenkessels (10) angeordneten Innenkessel (20) und mit einem Dampfeinlass (50), der eine Rückwand (14) des Außenkessels (10) durchsetzt, bei dem vor dem Dampfeinlass (50) im Bereich zwischen der Rückwand (14) des Außenkessels (10) und einer Innenkesselrückwand (24) eine Strömungsbarriere (51) angeordnet ist.

## Beschreibung

Klimaschränke werden allgemein verwendet, um Dinge bei definierter Temperatur und Luftfeuchtigkeit zu lagern. Um die Luftfeuchtigkeit erhöhen zu können, wenn dies notwendig ist, wird dabei üblicherweise durch einen Dampfeinlass Dampf in den Innenraum des Klimaschranks eingeleitet.

Dieser Dampfeinlass wird oft mittig zwischen den Seitenwänden des Klimaschranks in dessen Bodenbereich angeordnet und durchsetzt die im Bereich der Rückseite, d.h. der der Tür oder den Türen des Klimaschranks gegenüber liegenden Seite, platziert, um so zu versuchen, eine gleichmäßige Verteilung der Feuchtigkeit zu ermöglichen. Der zugeführte Dampf soll sich dann zwischen Innen- und Außenkessel ausbreiten und durch Öffnungen in den Wänden des Innenkessels, insbesondere in dessen Seitenwänden, in das Innere des Innenkessels eintreten.

Allerdings zeigt die Praxis, dass dabei nur in beschränktem Maße eine gleichmäßige Verteilung erreicht wird. Typischerweise breitet sich in der Mitte des Klimaschranks ausgehend vom mittig angeordneten Dampfeinlass ein sehr feuchter Luftstrom mit Dampfschwaden aus, der nur allmählich in die Sei-tenteile des Klimaschranks gelangt und eine signifikante Wahrscheinlichkeit für eine unerwünschte Betauung im Türbereich mit sich bringt.

Die Aufgabe der Erfindung besteht daher darin, einen Klimaschrank mit einer verbesserten Verteilung der durch seinen Dampfeinlass zugeführten Feuchtigkeit bereitzustellen. Diese Aufgabe wird gelöst durch einen Klimaschrank mit den Merkmalen des Patentanspruchs 1. Vorteilhafte Weiterbildungen der Erfindung sind Gegenstand der abhängigen Ansprüche.

Der erfindungsgemäße Klimaschrank hat einen Außenkessel, eine innerhalb des Außenkessels angeordneten Innenkessel und mit einem Dampfeinlass, der eine Rückwand des Außenkessels durchsetzt. Vorzugsweise wird der Dampfeinlass aus einem dem Klimaschrank zugeordneten, insbesondere zum Klimaschrank gehörenden Dampferzeuger gespeist, mit dem er durch ein Leitungssystem verbunden ist.

Erfindungswesentlich ist, dass vor dem Dampfeinlass im Bereich zwischen der Rückwand des Außenkessels und der Innenkesselrückwand eine Strömungsbarriere angeordnet ist. Dementsprechend ist die Strömungsbarriere nicht unmittelbar vor dem Dampfeinlass angeordnet, sie befindet sich aber aus Richtung der Tür des Klimaschranks gesehen weiter von dieser Tür entfernt als die Innenkesselrückwand und näher an dieser Tür als die Rückwand des Außenkessels.

Eine in diesem Bereich angeordnete Strömungsbarriere bewirkt, dass der Dampfstrahl nicht ungehindert im Wesentlichen entlang seiner Vorwärtsrichtung in den Innenraum des Klimaschranks und damit auf die Tür zu propagieren kann, sondern zunächst auf die Strömungsbarriere prallt und von dieser umgelenkt und/oder verwirbelt wird. Dadurch werden die Durchmischung des Dampfes mit der Luft und seine Verteilung in der Luft signifikant verbessert.

Besonders einfach kann eine solche Strömungsbarriere realisiert werden, wenn die Strömungsbarriere quaderförmig ist. Dann kann beispielsweise eine Platte aus einem geeigneten Material, beispielsweise bevorzugt aus demselben Material wie dem des Innenkessels, als Strömungsbarriere verwendete werden. Zudem ist die Installation einer quaderförmigen Strömungsbarriere sehr einfach; sie kann einfach parallel zur Rückwand des Außenkessels ausgerichtet -bevorzugt auf dem Boden des Außenkessels- befestigt werden.

Gemäß einer vorteilhaften Ausführungsform der Erfindung ist vorgesehen, dass die Höhe der Strömungsbarriere dem Abstand zwischen dem Boden des Außenkessels und dem Innenkesselboden entspricht und dass die Strömungsbarriere auf dem Boden des Außenkessels angeordnet ist. Auf diese Weise wird ein direkter Strom von wegen hohen Dampfgehaltes sehr feuchter Luft in Richtung auf die Tür des Klimaschranks hin effektiv verhindert, selbst wenn der Dampf relativ ungerichtet aus dem Dampfeinlass austritt.

Besonders bevorzugt ist es darüber hinaus, wenn der Klimaschrank mindestens einen Lüfter zum Ansaugen von Luft, insbesondere aus dem Innenraum des Klimaschranks, aufweist. Mit Hilfe eines solchen Lüfters kann eine Umwälzung der Luft im Innenraum des Klimaschranks bewirkt werden, die eine Verteilung des Dampfs fördert.

Wenn der Klimaschrank eine Verdampferplatte aufweist, die im Bereich zwischen der Innenkesselrückwand und der Rückwand des Außenkessels angeordnet ist, kann diese zu einer gezielten Regulierung der Feuchtigkeit im Klimaschrank beitragen.

Bei Klimaschränken mit Lüfter und Verdampferplatte kann es vorteilhaft sein, die Verdampferplatte derart unterhalb des mindestens einen Lüfters im Bereich zwischen der Innenkesselrückwand und der Rückwand des Außenkessels anzuordnen, dass von dem mindestens einen Lüfter angesaugte Luft sowohl zwischen der Innenkesselrückwand und der Verdampferplatte als auch zwischen der Verdampferplatte und der Rückwand des Außenkessels entlanggeführt wird.

Dadurch entstehen zwei Luftströme, die zunächst unterschiedliche Feuchtigkeitsgrade aufweisen. Der Luftstrom, der im Luftkanal zwischen der Verdampferplatte und der Rückwand des Außenkessels entlanggeführt wird, wird stark mit dem Dampf vermischt, der auf die Strömungsbarriere getroffen und durch diese verwirbelt ist und erhält dadurch einen relativ hohen Feuchtegrad. Dieser feuchte Luftstrom wird auf beiden Seiten seitlich an der Strömungsbarriere vorbei und unter der Verdampferplatte hindurch gelenkt und dadurch unmittelbar in die Seitenbereiche des Klimaschranks geführt, von wo er insbesondere durch Öffnungen in den Seitenwänden des Innenkessels in den eigentlichen Innenraum des Klimaschranks gelangt und den gewünschten Feuchtegrad einstellt.

Im Gegensatz dazu wird der Luftstrom, der im Luftkanal zwischen der Verdampferplatte und der Innenkesselrückwand entlanggeführt wird, kaum mit Dampf vermischt, sondern bildet einen relativ trockenen Luftvorhang aus. Im Mittelbereich des Klimaschranks gilt dies auch im Bereich, der dem Dampfeinlass gegenüber liegt, weil dieser Bereich durch die Strömungsbarriere vom Dampf abgeschirmt wird. Da in den Seitenbereichen des Klimaschranks bereits der durch die offenen Bereiche zwischen den Rändern der Strömungsbarriere und den Seitenwänden des Außenkessels kanalisierte feuchte Luftstrom strömt, wird die trockene Luft in Richtung auf die Tür weitergeleitet, an der dementsprechend die Taubildung besonders effektiv vermieden wird.

Dieser Effekt wird dadurch vergrößert, dass die Verdampferplatte unmittelbar an die Strömungsbarriere angrenzt und/oder dass die Verdampferplatte mit der Strömungsbarriere fluchtet.

Die Erfindung wird nachfolgend anhand von Figuren, die Ausführungsbeispiele zeigen, näher erläutert. Es zeigt:
- Fig. 1:: Eine Außenansicht eines Klimaschranks,
- Fig. 2:: einen Blick auf einen Ausschnitt eines Querschnitts durch den Klimaschrank, der entlang der senkrecht auf der Rückwand des Klimaschranks stehende Mittelebene A des Klimaschranks verläuft,
- Fig. 3:: einen Blick auf einen Querschnitt durch den Klimaschrank, der entlang der eine parallel zum Boden des Klimaschranks verlaufende Ebene B verläuft, wobei der Querschnitt den Klimaschrank mit entferntem Boden des Innenkessels zeigt, und
- Fig. 4:: einen Blick auf einen Querschnitt durch den Klimaschrank, der entlang einer parallel zur Rückwand des Klimaschranks verlaufenden Ebene C, die hinter der Rückwand des Innenkessels liegt.

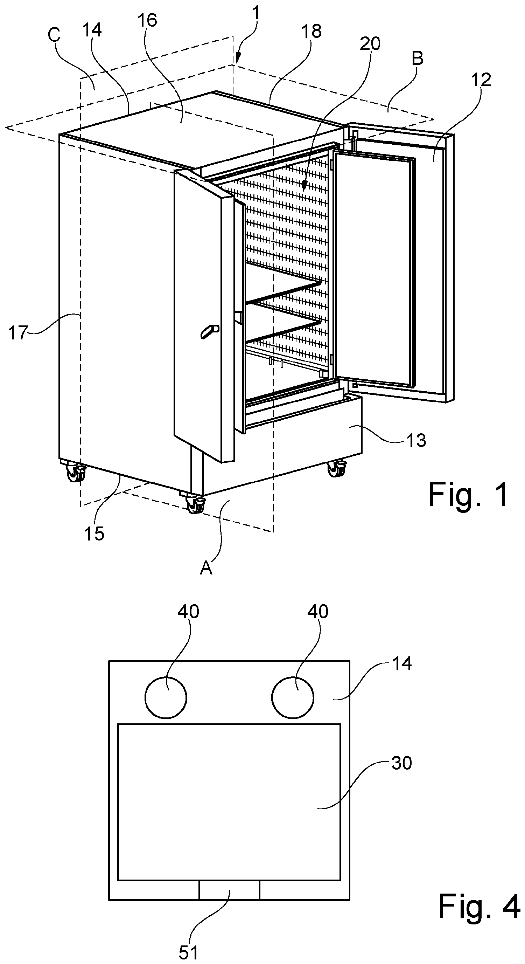

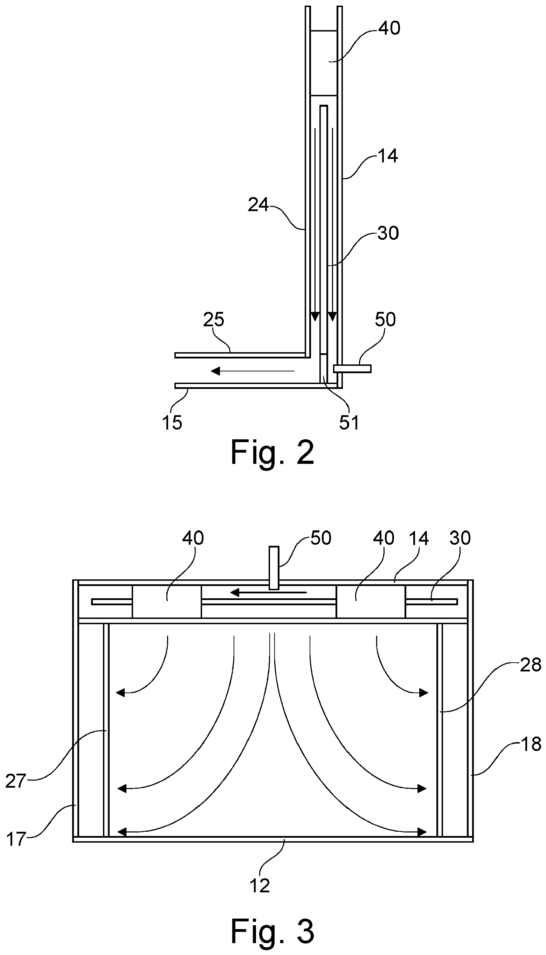

Figur 1 zeigt eine Außenansicht eines Klimaschranks 1. Der Klimaschrank 1 ist im wesentlichen quaderförmig, wobei die Außenflächen des Quaders, die zusammen den Außenkessel 10 bilden durch die durch die darin angeordnete Tür 12 identifizierbare Vorderwand 13, die der Vorderwand 13 gegenüber liegende Rückwand 14, einen Boden 15, eine Decke 16 sowie durch linke Wandfläche 17 und rechte Wandfläche 18 des Klimaschranks gebildet werden. Figur 1 zeigt darüber hinaus drei Schnittebenen A,B und C, die veranschaulichen, welche Ansichten die nachfolgend beschriebenen Figuren 2 bis 4 zeigen.

Der Klimaschrank 1 weist außer dem Außenkessel 10 auch noch einen Innenkessel 20 auf, der zumindest eine Innenkesselrückwand 24, einen Innenkesselboden 25 sowie Innenkesselseitenwände 27,28 hat, wie man insbesondere in den Figuren 2 und 3 erkennt.

Wie die Zusammenschau der Figuren 2 bis 4 zeigt, ist zwischen der Rückwand 14 des Außenkessels 10 und der Innenkesselrückwand 24 eine Verdampferplatte 30 vorgesehen, welche im Wesentlichen parallel zu diesen Wänden angeordnet ist. Der untere Rand der Verdampferplatte 30 endet in diesem Ausführungsbeispiel etwa auf der Höhe des Innenkesselbodens 25. Zudem überragt in diesem Ausführungsbeispiel die Verdampferplatte 30 die Innenkesselweitenwände 27,28 jeweils zur Seite hin.

Oberhalb der Verdampferplatte 30 sind zwei Lüfter 40 vorhanden, mit denen ein im Wesentlichen in Richtung auf den Boden 15 hin gerichteter Luftstrom erzeugt werden kann, der in Figur 2 durch Pfeile veranschaulicht wird. Die dazu benötigte Luft wird vorzugsweise zumindest teilweise aus dem Innenraum des Klimaschranks durch Öffnungen in der Innenkesselrückwand 24 angesaugt.

In der Nähe des Bodens 15 des Außenkessels 10, genauer gesagt im Bereich zwischen dem Boden 15 des Außenkessels 10 und dem Innenkesselboden 25, ist ein Dampfeinlass 50 in der Rückwand 14 des Außenkessels 10 vorhanden. Vor dem Dampfeinlass 50, aber von diesem beabstandet, ist eine Strömungsbarriere 51, die in diesem Beispiel durch eine quaderförmige Platte gebildet wird, angeordnet, und zwar so, dass sie mit der Verdampferplatte 30 fluchtet. Dazu ist die Dicke der Strömungsbarriere 51 an die Dicke der Verdampferplatte 30 angepasst und die Strömungsbarriere 51 ist in demselben Abstand zur Rückwand 14 des Außenkessels 10 angeordnet wie die Verdampferplatte 30.

Die Höhe der Strömungsbarriere 51, die sich in Richtung zum Boden 15 des Außenkessels 10 hin unmittelbar an die Verdampferplatte 30 anschließt, entspricht im Wesentlichen dem Abstand zwischen Innenkesselboden 25 und Boden 15 des Außenkessels 10.

Die Breite der Strömungsbarriere 51 ist, wie man insbesondere in Figur 4 erkennt, kleiner als der Abstand der Lüfter 40 voneinander und insbesondere kleiner als der Abstand der linken Wandfläche 17 des Außenkessels 10 zu seiner rechten Wandfläche 18. Dementsprechend kann Luft, die zwischen der Verdampferplatte 30 und der Rückwand 14 des Außenkessels 10 strömt, rechts und links neben der Strömungsbarriere 51 austreten.

Wie Figur 4 zeigt, ist die Strömungsbarriere 51 in etwa mittig angeordnet. Sie kann aber auch mit einem Versatz angeordnet werden, was insbesondere dann sinnvoll sein kann, wenn ein die Lüfter 40 dem Luftstrom einen Drall aufprägen.

Insbesondere die Darstellung gemäß Figur 2 verdeutlicht schematisch den Strömungsverlauf von durch Lüfter 40 in den Raum zwischen der Innenkesselrückwand 24 und der Rückwand 14 des Außenkessels 10 geförderten Luft. Diese strömt vor und hinter der Verdampferplatte 30 in Richtung auf den Boden 15 des Außenkessels 10 nach unten. Insbesondere im Strömungskanal zwischen Rückwand 14 des Außenkessels 10 und Verdampferplatte 30 kommt es zu einer intensiven Vermischung mit durch den Dampfeinlass 50 eingespeistem Dampf, da der eingespeiste Dampfstrahl nach seinen Eintritt in den Raum zwischen Innenkesselrückwand 24 und Rückwand 14 des Außenkessels 10 auf die Strömungsbarriere 51 trifft und verwirbelt wird. Insbesondere verhindert die Strömungsbarriere 51 damit die direkte Propagation von Dampf in Richtung auf die Tür 12 hin und reduziert damit die Problematik einer Betauung im Türbereich.

Ein weiterer wünschenswerter Einfluss der Strömungsbarriere 51 ergibt sich daraus, dass die mit dem Dampf vermischte Luft im Wesentlichen seitlich an der Strömungsbarriere 51 vorbeigeführt wird. Insbesondere dann, wenn diese Luft über Öffnungen in den Seitenwänden 27,28 des Innenkessels 20 in den für die Lagerung vorgesehenen Innenraum des Klimaschranks 1 geführt werden soll, wird der Strom von mit Dampf vermischter Luft auf diese Weise relativ direkt zu den Stellen geführt, wo dieser Eintritt erfolgen soll.

Im Gegensatz zu der Luft, die auf der Rückseite der Verdampferplatte 30 entlang strömt, kommt es bei der Luft, die im Strömungskanal zwischen der Innenkesselrückwand 24 und der Verdampferplatte 30 strömt, kaum zu Wechselwirkung mit Dampf, da der direkte Zugang des Dampfes zu diesem Bereich durch die Verdampferplatte 30 und die Strömungsbarriere 51 blockiert wird. Dadurch wird insbesondere im Mittelbereich ein Schleier trockener Luft erzeugt, welcher in Richtung auf die Tür 12 hin transportiert wird und eine Betauung der Tür 12 sicher verhindert.

### Bezugszeichenliste

- 1: Klimaschrank
- 10: Außenkessel
- 12: Tür
- 13: Vorderwand
- 14: Rückwand
- 15: Boden
- 16: Decke
- 17: linke Wandfläche
- 18: rechte Wandfläche
- 20: Innenkessel
- 24: Innenkesselrückwand
- 25: Innenkesselboden
- 27,28: Innenkesselseitenwand
- 30: Verdampferplatte
- 40: Lüfter
- 50: Dampfeinlass
- 51: Strömungsbarriere

- A,B,C: Schnittebene

## Patentansprüche

1. Klimaschrank (1) mit einem Außenkessel (10), mit einem innerhalb des Außenkessels (10) angeordneten Innenkessel (20) und mit einem Dampfeinlass (50), der eine Rückwand (14) des Außenkessels (10) durchsetzt **dadurch gekennzeichnet, dass** vor dem Dampfeinlass (50) im Bereich zwischen der Rückwand (14) des Außenkessels (10) und einer Innenkesselrückwand (24) eine Strömungsbarriere (51) angeordnet ist.

2. Klimaschrank (1) nach Anspruch 1, **dadurch gekennzeichnet, dass** die Strömungsbarriere quaderförmig ist.

3. Klimaschrank (1) nach Anspruch 1 oder 2, **dadurch gekennzeichnet, dass** die Höhe der Strömungsbarriere (51) dem Abstand zwischen dem Boden (15) des Außenkessels (10) und dem Innenkesselboden (25) entspricht und dass die Strömungsbarriere (51) auf dem Boden (15) des Außenkessels angeordnet ist.

4. Klimaschrank (1) nach einem der Ansprüche 1 bis 3, **dadurch gekennzeichnet, dass** der Klimaschrank (1) mindestens einen Lüfter (40) zum Ansaugen von Luft, insbesondere aus dem Innenraum des Klimaschranks (1), aufweist.

5. Klimaschrank (1) nach einem der Ansprüche 1 bis 4, **dadurch gekennzeichnet, dass** der Klimaschrank eine Verdampferplatte (30) aufweist, die im Bereich zwischen der Innenkesselrückwand (24) und der Rückwand (14) des Außenkessels (10) angeordnet ist.

6. Klimaschrank (1) nach Anspruch 4 und 5, **dadurch gekennzeichnet, dass** die Verdampferplatte (30) derart unterhalb des mindestens einen Lüfters (40) im Bereich zwischen der Innenkesselrückwand (24) und der Rückwand (14) des Außenkessels (10) angeordnet ist, dass von dem mindestens einen Lüfter (40) angesaugte Luft sowohl zwischen der Innenkesselrückwand (24) und der Verdampferplatte (30) als auch zwischen der Verdampferplatte (30) und der Rückwand (14) des Außenkessels (10) entlanggeführt wird.

7. Klimaschrank (1) nach einem der Ansprüche 5 oder 6, **dadurch gekennzeichnet, dass** die Verdampferplatte (30) unmittelbar an die Strömungsbarriere (51) angrenzt.

8. Klimaschrank (1) nach einem der Ansprüche 5 bis 7, **dadurch gekennzeichnet, dass** die Verdampferplatte (30) mit der Strömungsbarriere (51) fluchtet.
